# EUROPÄISCHE PATENTSCHRIFT

(11) **EP 0 443 198 B1**
(45) Veröffentlichungstag und Bekanntmachung des Hinweises auf die Patenterteilung: **03.01.1996**
(21) Anmeldenummer: 90125409.4
(22) Anmeldetag: 24.12.1990
(51) Int. Cl.: C07H 15/203

(54) **Diterpene mit immunmodulatorischer Wirkung**
Diterpenes with immunomodulatory activity
Diterpènes avec de l'activité immunomodulatrice

(30) Priorität: 17.02.1990 DE 4005159
(43) Veröffentlichungstag der Anmeldung: 28.08.1991
(73) Patentinhaber: MERCK PATENT GmbH, D-64271 Darmstadt (DE)
(72) Erfinder: Radunz, Hans-Eckart, Dr., W-6109 Mühltal (DE); Wolf, Michael, Dr., W-6100 Darmstadt (DE); Baumgarth, Manfred, Dr., W-6100 Darmstadt (DE); Kinzy, Willy, Dr., W-6845 Gross-Rohrheim (DE); Luckenbach, Gerd-Albrecht, Dr., W-6100 Darmstadt (DE)

(56) Entgegenhaltungen:
- FITOTERPIA Bd. LVI, Nr. 4, 1985, MILAN (IT) Seiten 249 - 250; RAMACHANDRAN NAIR ET AL: 'POLYPHENOLIC COMPOUNDS FROM LEAVES OF ACANTHOSPERMUM HISPIDUM'
- PHYTOCHEMISTRY Bd. 29, Nr. 1, Januar 1990, OXFORD (UK) Seiten 321 - 323; A.G.GONZALEZ ET AL: 'DITERPENES AND OTHER CONSTITUENTS OF EUPATORIUM SALVIA'
- PHYTOCHEMISTRY Bd. 27, Nr. 2, Februar 1988, OXFORD (UK) Seiten 501 - 504; J.G.URONES ET AL: 'DITERPENOIDS OF HALIMIUM VISCOSUM'

## Beschreibung

Die Erfindung betrifft Diterpenderivate, die eine immunmodulatorische, insbesondere die T-Zellen stimulierende Wirkung aufweisen und somit als pharmazeutische Wirkstoffe geeignet sind.

Das Ausbrechen von Krankheiten bedeutet letztlich, daß das Immunsystem des betreffenden Individiums in bestimmter Weise zumindest vorübergehend angegriffen und geschwächt worden ist. Neben der ganz gezielten medikamentösen Behandlung von meist durch spezifische Erreger verursachte Krankheiten kommt der allgemeinen Stärkung der Immunabwehr eine wichtige Rolle zu, insbesondere seitdem man festgestellt hat, daß auch die Entstehung von Tumoren und von Autoimmunkrankheiten zum Teil durch allgemeiner wirkende immunmodulatorische Substanzen verhindert oder in positiver Weise beeinflußt werden kann. Auf der Suche nach entsprechend geeigneten Substanzen bedient sich die pharmazeutische Industrie unter anderem im zunehmenden Maß verschiedenartiger Naturstoffe, zumeist in Form von undefinierten Extrakten oder Essenzen. Bekannte Beispiele hierfür sind die als Immunstimulanzen wirkenden und im Handel erhältlichen pflanzlichen Extrakte aus Sonnenhut (Echinacea) oder Thuja. Alle derartigen Extrakte haben den Nachteil, daß die eigentlichen Wirkstoffe in der Regel nicht bekannt sind und zudem nur in sehr geringen Konzentrationen vorliegen, ihre Isolierung und die Identifizierung der meist komplizierten chemischen Strukturen nicht unerhebliche Schwierigkeiten bereiten, die Wirkung zum Teil unspezifisch ist und nicht selten eine Kombinationswirkung darstellt, oder der Extrakt als solcher in zahlreichen Fällen toxisch ist.

Es bestand somit die Aufgabe neue pharmazeutische Wirkstoffe zur Verfügung zu stellen, die zur Stärkung der Immunabwehr eingesetzt werden können und die sich aus geeigneten pflanzlichen Naturstoffpräparaten in eindeutiger Weise isolieren, charakterisieren und chemisch modifizieren lassen, so daß ein in seinen Eigenschaften, seiner Struktur und seiner pharmakologischen Wirkungsweise wohl definiertes Arzneimittel bereitgestellt werden kann.

Es wurde nun gefunden, daß bestimmte Diterpenderivate beispielsweise aus Pflanzen der Gattungen Acanthospermum, Brickellia, Halimium oder Ayapana sowie ihre nach Isolierung chemisch modifizierten Varianten eine überraschend stark ausgeprägte immunmodulatorische Wirkung zeigen. Insbesondere ist eine signifikante Stimulierung der T-Zellen, die bei der Immunabwehr eine wichtige Rolle spielen, zu beobachten. Die erfindungsgemäßen Wirkstoffe sind innerhalb des Dosierungsbereichs nicht toxisch, wirken nicht mitogen auf Lymphozyten und haben auch keinen negativen Einfluß auf das zentrale Nervensystem. Sie eignen sich also vorzüglich für den Einsatz in der Immuntherapie.

Gegenstand der Erfindung sind somit Arzneimittel der Formel I, 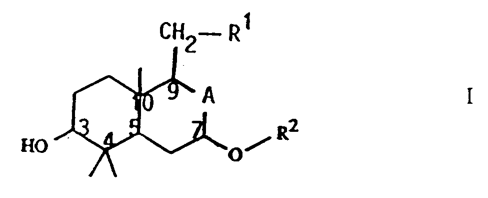 worin
- R¹: -CH₂-C(CH₃)=CH-R³ oder -CH₂-CH(CH₃)-CH₂-R³,
- R³: -CH₂OH, -CHO, -COOH oder -COOR⁴,
- R⁴: Alkyl mit 1 bis 5 C-Atomen,
- A: 〉CH-CH₃

oder

〉C=CH₂

und
- R: Pentosen, Hexosen, Disaccharide, Glucosamin oder Galactosamin bedeuten.

Gegenstand der Erfindung ist insbesondere der pharmazeutische Wirkstoff der Formel Ia

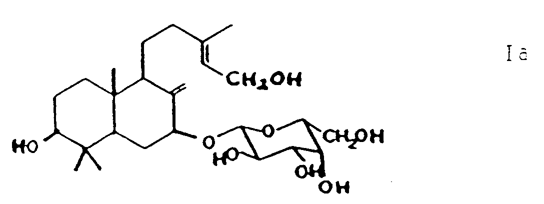

Gegenstand der Erfindung ist weiter eine pharmazeutische Zubereitung, dadurch gekennzeichnet, daß sie mindestens eine Verbindung der Formel I und/oder eines ihrer physiologisch unbedenklichen Säureadditionssalze enthält.

Außerdem ist Gegenstand der Erfindung ein Verfahren zur Herstellung pharmazeutischer Zubereitungen, dadurch gekennzeichnet, daß man eine Verbindung der Formel I und/oder eines ihrer physiologisch unbedenklichen Säureadditionssalze zusammen mit mindestens einem festen, flüssigen oder halbflüssigen Träger- oder Hilfsstoff in eine geeignete Dosierungsform bringt.

Gegenstand der Erfindung sind ferner die neuen Verbindungen der Formel II 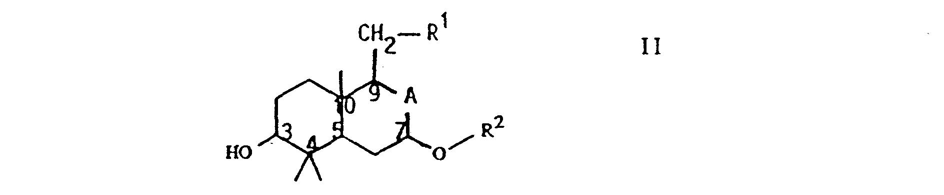 worin
- R¹: -CH₂-C(CH₃)=CH-R³ oder -CH₂-CH(CH₃)-CH₂-R³,
- R³: -CH₂OH, -CHO, -COOH oder -COOR⁴,
- R⁴: Alkyl mit 1 bis 5 C-Atomen,
- A: 〉CH-CH₃

oder

〉C=CH₂

und
- R: Pentosen, Hexosen, Disaccharide, Glucosamin oder Galactosamin bedeuten,
mit der Maßgabe, daß nicht gleichzeitig R¹ -CH₂-C(CH₃)=CH-CH₂OH oder -CH₂-C(CH₃)=CH-CHO, A

〉C=CH₂

und R Galactose bedeuten darf.

Gegenstand der Erfindung ist weiterhin ein Verfahren zur Herstellung der Verbindungen der Formel II, dadurch gekennzeichnet, daß man wahlweise
(a) die Verbindung der Formel Ia aus Pflanzen der Gattung Acanthospermum isoliert und aufreinigt und mindestens einen der folgenden Syntheseschritte durchführt:
   - Hydrierung der trisubstituierten Doppelbindung,
   - Hydrierung der exocyclischen Doppelbindung,
   - Oxidation der -CH₂OH-Gruppe des Diterpenanteils zum Aldehyd oder zur Carbonsäure, bzw. zum Carbonsäureester,
   - Substitution des Galactoserestes durch einen anderen Zucker,
(b) die Verbindung der Formel III 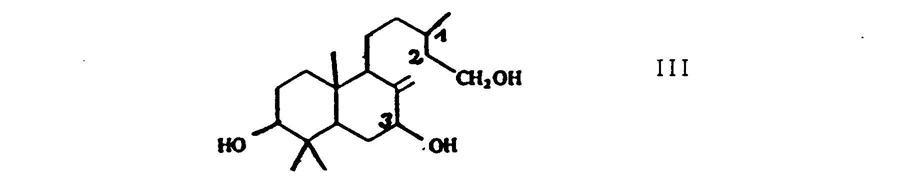 aus Pflanzen der Gattungen Brickellia oder Halimium isoliert und aufreinigt und mindestens einen der folgenden Syntheseschritte durchführt:
   - Dehydrierung der C1-C2 Bindung der Formel III,
   - Hydrierung der exocyclischen Doppelbindung,
   - Oxidation der -CH₂OH-Gruppe zum Aldehyd oder zur Carbonsäure, bzw. zum Carbonsäureester,
   - Glykosidierung der Hydroxylgruppe am C3-Atom,
   oder
   (c) die Verbindung der Formel IV 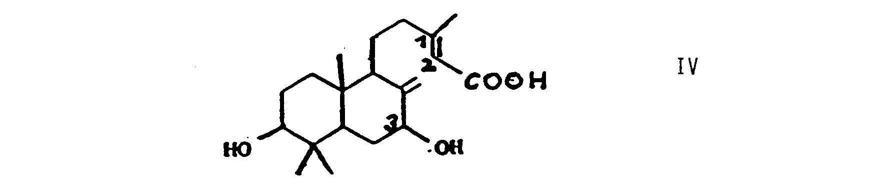 aus Pflanzen der Gattung Ayapana isoliert und aufreinigt und mindestens einen der folgenden Syntheseschritte durchführt:
   - Hydrierung der trisubstituierten Doppelbindung,
   - Hydrierung der exocyclischen Doppelbindung,
   - Veresterung der Carbonsäure,
   - Reduktion der Carbonsäure oder des Carbonsäureesters zum Aldehyd oder Alkohol
   - Glykosidierung der Hydroxylgruppe am C3-Atom.

Gegenstand der Erfindung ist letztlich die Verwendung der Verbindungen der Formel I zur Herstellung von Arzneimitteln mit immunmodulatorischer, insbesonderer T-Zellen stimulierender Wirkung.

Die Verbindungen der Formel I als solche sind teilweise bekannt. Insbesondere sind die Verbindungen der Formeln Ia, III und IV bekannt. Ihre therapeutische Wirkung hingegen ist neu. Die Verbindungen der Formeln Ia, III und IV lassen sich beispielsweise in bekannter Weise aus Pflanzen bzw. Pflanzenteilen der Gattungen Acanthospermum, Brickellia, Halimium oder Ayapana gewinnen. Sie wurden bezüglich ihrer Struktur hinreichend charakterisiert (Nair et al. (1976), Phytochemistry 15, 1776; Bohlmann et al. (1979), Phytochemistry 18, 1997; de Pascual Teresa et al. (1985), Phytochemistry 24, 791; Achmed et al. (1986), Phytochemistry 25, 1385). Die genannten Pflanzen sind wild wachsende Pflanzen aus tropischen und subtropischen aber auch aus gemäßigt warmen Zonen, wie beispielsweise Süd- und Mittelamerika, Indien oder mediterrane Gegenden. Die Isolierung und Aufreinigung der Verbindungen Ia, III und IV am Blatt- oder Wurzelsegmenten ist in den genannten Literaturzitaten ausreichend beschrieben.

Bevorzugte Arten sind: Acanthospermum Hispidum, Ayapana Amygdalina, Halimium Viscosum und Brickellia Vernicosa.

Die neuen Verbindungen der Formel II, die ebenfalls immunmodulatorische, bzw. immunstimulierende Wirkung zeigen, lassen sich beispielsweise aus den aufgereinigten Naturstoffen der Formeln Ia, III und IV in an sich bekannter Weise nach Standardmethoden der organischen Chemie herstellen.

So läßt sich beispielsweise die trisubstituierte Doppelbindung der Verbindungen der Formeln Ia, II und IV in üblicher Weise katalytisch hydrieren, vorzugsweise aber mit Platin in Essigsäure.

Die Hydrierung der exocyclischen Doppelbindung (A:

〉C=CH₂

zu

〉CH-CH₃)

wird vorzugsweise ebenfalls katalytisch durchgeführt. Insbesondere eignet sich hier Palladium auf Aktivkohle in Methanol, aber auch andere Katalysatoren können eingesetzt werden.

Die Oxidation der allylischen -CH₂OH-Gruppen, beispielsweise in den Verbindungen der Formel Ia, II und III zu den entsprechenden Allylaldehyden geschieht vorzugsweise mit aktivem Braunstein in an sich bekannter Weise. Ist die allylische Doppelbindung bereits hydriert, so kann die Oxidation des primären Alkohols am Diterpenrest zum Aldehyd auch mit hierfür anderen gängigen Oxidationsmitteln durchgeführt werden, vorzugsweise aber mit Kupfer, Kupferchromit oder Silber, da hier die Weiteroxidation zur Carbonsäure gehemmt ist.

Die Oxidation des betreffenden primären Alkohols bzw. des Aldehyds zur Carbonsäure geschieht in einfacher und bekannter Weise z.B. mit KMnO₄, CrO₃, K₂Cr₂O₇ in Pyridin.

Die Veresterung des -COOH Restes mit den Alkoholen des Typs R⁴OH geschieht beispielsweise säure- oder basenkatalysiert nach Standardverfahren. Auch durch Umesterungen läßt sich der gewünschte Rest R⁴ einführen.

Die Glykosidierung der C3-Hydroxylgruppe der Verbindungen der Formeln III und IV bzw. der C7-Hydroxylgruppe der Verbindungen der Formel I (R = H) wird nach Standardmethoden der Kohlenhydratchemie durchgeführt. Vorzugsweise wird der Zuckerrest mittels Fischer-,Helferich oder der Koenigs-Knorr-Synthese an den Diterpenbaustein ansynthetisiert. Bei den Reaktionen entstehen α und β-Glykoside. Durch bestimmte Verfahrensvarianten können beispielsweise β-Glykoside, die vorzugsweise nach der Koenigs-Knorr-Synthese hergestellt werden, durch Behandlung mit z.B. Titantetrachlorid in apolaren Lösungsmitteln anomerisiert und somit fast quantitativ in die entsprechenden α-Glykoside überführt werden. Erfindungsgemäß sind sowohl die α- als auch die β-Glykoside geeignet. Die Zuckeranknüpfung läßt sich auch besonders vorteilhaft mit der Imidatmethode durchführen (R. Schmidt (1986), Angewandte Chemie 98, 213).

Erfindungsgemäß bedeutet R in den Verbindungen der Formeln I und II Pentosen, Hexosen, Disaccharide, Glucosamin oder Galactosamin. Im Vergleich zu den Zuckerfreien Verbindungen besitzen die entsprechenden Glykoside in der Regel die bessere Wirkstoffeigenschaft im Sinne einer T-Zellen stimulierenden Wirkung und Weisen insbesondere im Falle von Mono-/Disacchariden eine bessere Löslichkeit auf. Unter den Zuckern sind die Monosaccharide bevorzugt. Hierunter fallen beispielsweise Pentosen wie Ribose, Threose oder Xylose, Hexosen wie Glucose, Mannose, Fructose oder Galactose oder aber auch die Aminozucker Glucosamin und Galactosamin. Erfindungsgemäß wird Galactose besonders bevorzugt. Als Disaccharide sind vor allem Lactose, Maltose oder Cellobiose zu nennen.

Erfindungsgemäß bedeutet R¹ -CH₂-C(CH₃)=CH-R³ oder -CH₂-CH(CH₃)-CH₂-R³ worin R³ -CH₂OH, -CHO, -COOH oder -COOR⁴ bedeutet. Besonders bevorzugte Reste R¹ sind -CH₂-C(CH₃)=CH-CH₂OH,-CH₂-C(CH₃)=CH-CHO, -CH₂-C(CH₃)=CH-COOH, -CH₂-CH(CH₃)-CH₂-CH₂OH und -CH₂-CH(CH₃)-CH₂-COOH. Innerhalb der Ester -COOR⁴ bedeutet R⁴ vorzugsweise Methyl, Ethyl, n-Propyl, n-Butyl, iso-Propyl, tert.-Butyl oder 2-Methylpropyl.

Die bevorzugte Bedeutung von A ist

〉C=CH₂.

In den besonders bevorzugten Verbindungen der Formeln I oder II bedeutet
- R¹: -CH₂-C(CH₃)=CH-CH₂OH oder -CH₂-C(CH₃)=CH-CHO,
- A: 〉C=CH₂

und
- R: Galactose, Glucose, Fructose oder Ribose, insbesondere aber Galactose.

Die erfindungsgemäßen Verbindungen weisen im Diterpenteil mehrere asymmetrische C-Atome auf, die eine entsprechende Stereospezifität bedingen. Hierbei sind insbesondere die Positionen 3, 7, 9 und 10 der Verbindungen der Formeln I und II von Bedeutung, da diese in den verschiedenen Naturstoffpräparationen zuweilen unterschiedlich sind. So liegt beispielsweise in Acanthospermum Hispidum eine 3R, 7R, 9S, 10R-Konfiguration, in Ayapana Amygdalina eine 3R, 7R, 9R, 10S-Konfiguration und in Brickellia Vernicosa eine 3S, 7R, 9S, 10R-Konfiguration vor. Da die relative Konfiguration an den betreffenden Asymmetriezentren für die genannte pharmazeutische bzw. therapeutische Wirksamkeit der erfindungsgemäßen Arzneimittel keine essentielle Rolle spielt, sind aus Gründen der einfacheren Verfügbarkeit die Konfigurationen, die die Naturstoffe besitzen, bevorzugt. Selbstverständlich können erfindungsgemäß durch chemisch bedingte Isomerisierung nach Standardmethoden die entsprechend anders konfigurierten Verbindungen hergestellt werden.

Eine Base der Formeln I und II kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Umgekehrt können saure Verbindungen der Formel I durch Behandeln mit einer Base in eines ihrer Metall- (z. B. Na-, K-, Ca-) bzw. Ammoniumsalze umgewandelt werden.

Quartäre Ammoniumsalze der Verbindungen der Formel I und II, z.B. bei Aminozuckern, sind erhältlich durch Behandeln derselben mit Quaternisierungsmitteln, z.B. Alkyl- oder Aralkyl-halogeniden, -sulfonaten oder -sulfaten wie Methylchlorid, -bromid, -jodid, -p-toluolsulfonat, Dimethylsulfat, Ethylbromid, Benzylchlorid, zweckmäßig in Gegenwart eines inerten Lösungsmittels, z. B. eines Alkohols wie Methanol oder Ethanol, bei Temperaturen zwischen -10 und +30°.

Die erfindungsgemäßen Verbindungen der Formel I, Ia und II und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z. B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z. B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabfolgt werden. Die neuen Verbindungen können auch lyophilisiert und die erhaltenen Lyophilisate z. B. zur Herstellung von Injektionspräparaten verwendet werden.

Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine.

Die parenterale Applikation ist bevorzugt.

Die immunmodulatorische Wirkung von menschlichen oder tierischen T-Lymphozyten in vitro kann zum Beispiel in folgender Weise gemessen und bestimmt werden. Die Methode beruht letztlich darauf, daß T-Zellen selektiv durch das Lektin Phytohämagglutinin (PHA) zur Mitose angeregt werden, ohne daß eine vorherige Abtrennung andere Lymphozyten, wie z.B. Makrophagen oder B-Zellen, notwendig ist. Die Stärke der T-Zellaktivierung ist dabei von der eingesetzten Menge PHA abhängig. Wählt man eine etwa halbmaximale Aktivierung von T-Zellen, so kann die Immunmodulation besonders gut gemessen werden. Immunmodulatorisch wirksame Substanzen, die dem Testansatz zugefügt werden,erhöhen (Immunstimulation) oder erniedrigen (Immunsuppression) die bereits vorhandene Zellteilung. Therapeutisch ungeeignete Substanzen bewirken dagegen ohne vorhergehende Aktivierung eine "ungerichtete" Zellteilung der T-Lymphozyten. Dies kann zu pathologischen Reaktionen des Immunsystems führen. Als indirektes Maß für die Zellteilung dient der Einbau bzw. die Messung von radioaktivem Thymidin in neusynthetisierte DNA in den Zellen.

Die erfindungsgemäßen Wirkstoffe bewirken eine signifikante Stimulierung von humanen T-Lymphozyten. So kann gegenüber dem Kontrollwert (ohne Wirkstoff) eine Steigerung der T-Zellen-aktivität von 30 bis 80 %, vorzugsweise von 40 - 65 % beobachtet werden. Die Verbindungen wirken nicht mitogen auf Lymphozyten.

Die glykosidischen Verbindungen zeigen generell eine höhere Wirksamkeit als die nicht glycosidierten. Die Substanzen sind generell gut verträglich. Auch negative Auswirkungen auf das zentrale Nervensystem können nicht festgestellt werden. Die erfindungsgemäßen Substanzen wurden vorzugsweise in Dosierungen zwischen etwa 0,1 mg und 10 mg, insbesondere zwischen 0,5 mg und 2,5 mg pro Dosierungseinheit verabreicht. Die tägliche Dosierung liegt vorzugsweise zwischen 0,1 und 1 mg/kg Körpergewicht. Die Dosis für jeden bestimmten Patienten hängt jedoch von den verschiedensten Faktoren ab, beispielsweise von der Wirksamkeit der angesetzten speziellen Verbindung, vom Alter, Körpergewicht, allgemeinen Gesundheitszustand, Geschlecht, von der Kost, von der Ausscheidungsgeschwindigkeit, von einer möglichen Arzneistoffkombination sowie von der Schwere der jeweiligen Erkrankung, der die Therapie gilt. Die erfindungsgemäßen Verbindungen sind auch zur Prophylaxe bestens geeignet.

### Beispiel 1

### Isolation menschlicher Lymphozyten aus dem peripherem Blut

Heparinisiertes Blut (10 units Lithiumheparinat/ml Blut) von normalen, gesunden Spendern wird mit dem gleichen Volumen eines für diese Zwecke geeigneten Kulturmediums (RPMI 1640, GIBCO, KA) verdünnt. 10 ml eines geeigneten handelsüblichen Gradientenmediums (z.B. Lymphoprep®, Dr. Molter GmbH, FRA) in einem 50 ml Schraubenverschlußröhrchen werden mit 20 ml verdünntem Blut langsam überschichtet. Der Gradient wird bei 700 x g 20 Minuten lang zentrifugiert. Dabei entstehen 3 Phasen: Die obere Phase wird abgesaugt und verworfen. Dann wird die scharfe Interphase mit den Lymphozyten und Monozyten/Makrophagen entnommen, in ein 15 ml Schraubenverschlußröhrchen überführt und auf 14 ml mit Kulturmedium aufgefüllt. Das Röhrchen wird bei 400 x g 15 Minuten lang zentrifugiert und der Überstand dekantiert. Es werden nochmals 10 ml Kulturmedium in das Röhrchen gegeben, die Zellen suspendiert und bei 275 x g für 10 Minuten zentrifugiert. Der Überstand wird dekantiert, das Pellet aufgelockert, in 2 ml Kulturmedium aufgenommen und darin die Zellen suspendiert. Eine Probe von 100 ul wird der Suspension entnommen und mit einer Trypanblaulösung (0,5 %) 1 : 10 verdünnt. Die Anzahl vitaler Zellen wird in einer Neubauer-Zählkammer bestimmt. Es werden mindestens 100 Zellen in mindestens 2 (gegenüberliegenden) Quadranten ausgezählt.

### Beispiel 2

### Mitogene Stimulierung

Die nach Beispiel 1 isolierten Lymphozyten werden auf 1 x 10⁶ Zellen/ml Kulturmedium eingestellt. Pro Vertiefung einer Mikrotiterplatte mit flachem Boden werden 0,2 ml Zellsuspension eingesetzt. Das Mitogen Phytohämagglutinin (PHA) wird zu jeder dieser Mikrokulturen hinzugefügt, so daß etwa 0.04 ug PHA/Kultur vorliegen. Die Menge ist so gewählt, daß eine etwa halb-maximale T-Zellen Aktivierung zu erwarten ist. Zu diesen Ansätzen wurden verschiedene Konzentrationen (50 - 500 ng/Kultur) der erfindungsgemäßen Wirkstoffe gegeben. Ein Ansatz dient als Kontrollwert.

### Beispiel 3

### Proliferation der Lymphozyten

Die Mikrotiterplatten mit den Kulturen gemäß Beispiel 2 werden 4 Tage lang in einem Brutschrank, der mit 10 % CO₂ begast wird, bei 37°C und 95 % relativer Luftfeuchtigkeit kultiviert. 18 Stunden vor Beendigung des Versuchs wurden die Kulturen mit 0,5 uCi ³H-Thymidin/Kultur versetzt. Anschließend werden die Kulturen weiter inkubiert. Am 4. Tag werden die Zellen mit einem Zellerntegerät auf einen Glasfaserfilter gesaugt. Die Filtermatte wird in eine Folie gegeben, mit 10 ml handelsüblicher Szinzillationsflüssigkeit getränkt und anschließend eingeschweißt. Die Messung der Radioaktivität von in DNA eingebautem ³H-Thymidin wird in einem handelsüblichen Betastrahlen-Szintillationsgerät vorgenommen.

### Beispiel 4

### Wirkung der Verbindung der Formel Ia

Die Verbindung wurde nach dem von Nair et al. angegebenen Verfahren isoliert und aufgereinigt (Phytochemistry (1976), 15, 1776). In dem Test gemäß den Beispielen 1 bis 3 zeigt sie bei einer Konzentration zwischen 100 - 200 mg/Kultur eine T-Zellen stimulierende Wirkung von 51 %, sowie keine Mitogenität auf Lymphozyten. Im in vivo ZNS-screening (nach Irwin (1968), Psychopharmacologica 13, 222) an der Maus kann bei Gaben von 0.5 mg/kg (i.v.) nach 1,4 und 24 Stunden keine substanzbedingte Wirkung festgestellt werden.

### Beispiel 5

### Wirkung der Verbindung der Formel Ia, worin der Allylalkohol durch -CHO ersetzt wurde

Die Verbindung wurde analog Beispiel 4 hergestellt und bezüglich ihrer immunstimulierenden Wirkung untersucht. Gegenüber dem Kontrollwert wurde eine Aktivitätssteigerung (T-Zellen) von 40 % gemessen.

## Patentansprüche (Patentansprüche für folgende(n) Vertragsstaat(en): DE, GB, FR, IT, NL, SE, CH, LU, BE, AT, DK)

1. Arzneimittel der Formel I 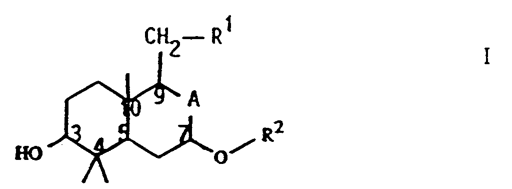 worin
R¹ -CH₂-C(CH₃)=CH-R³ oder -CH₂-CH(CH₃)-CH₂-R³,
R³ -CH₂OH, -CHO, -COOH oder -COOR⁴,
R⁴ Alkyl mit 1 bis 5 C-Atomen,
A
〉CH-CH₃
oder
〉C=CH₂
und
R Pentosen, Hexosen, Disaccharide, Glucosamin oder Galactosamin bedeuten.

2. Arzneimittel nach Anspruch 1, worin R Galactose ist.

3. Arzneimittel der Formel Ia

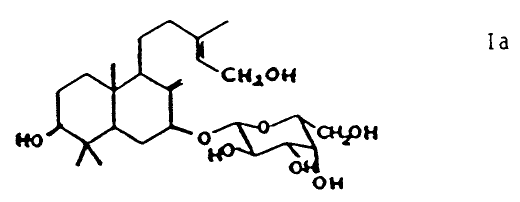

4. Pharmazeutische Zubereitung, dadurch gekennzeichnet, daß sie mindestens eine Verbindung der Formel I nach Anspruch 1, und/oder eines ihrer physiologisch unbedenklichen Säureadditionssalze enthält.

5. Verfahren zur Herstellung pharmazeutischer Zubereitungen, dadurch gekennzeichnet, daß man eine Verbindung der Formel I nach Anspruch 1, und/oder eines ihrer physiologisch unbedenklichen Säureadditionssalze zusammen mit mindestens einem festen, flüssigen oder halbflüssigen Träger- oder Hilfsstoff in eine geeignete Dosierungsform bringt.

6. Verbindungen der Formel II 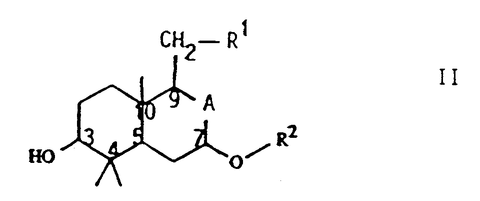 worin
R¹ -CH₂-C(CH₃)=CH-R³ oder -CH₂-CH(CH₃)-CH₂-R³,
R³ -CH₂OH, -CHO, -COOH oder -COOR⁴,
R⁴ Alkyl mit 1 bis 5 C-Atomen,
A
〉CH-CH₃
oder
〉C=CH₂
und
R Pentosen, Hexosen, Disaccharide, Glucosamin oder Galactosamin bedeuten,
mit der Maßgabe, daß nicht gleichzeitig R¹ -CH₂-C(CH₃)=CH-CH₂OH oder -CH₂-C(CH₃)=CH-CHO, A
〉C=CH₂
und R Galactose bedeuten darf.

7. Verfahren zur Herstellung der Verbindungen der Formel II gemäß Anspruch 6, dadurch gekennzeichnet, daß man wahlweise
(a) die Verbindung der Formel Ia aus Pflanzen der Gattung Acanthospermum isoliert und aufreinigt und mindestens einen der folgenden Syntheseschritte durchführt:
- Hydrierung der trisubstituierten Doppelbindung,
- Hydrierung der exocyclischen Doppelbindung,
- Oxidation der -CH₂OH-Gruppe des Diterpenanteils zum Aldehyd oder zur Carbonsäure, bzw. zum Carbonsäureester,
- Substitution des Galactoserestes durch einen anderen Zucker,
(b) die Verbindung der Formel III 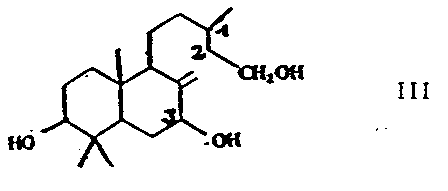 aus Pflanzen der Gattungen Brickellia oder Halimium isoliert und aufreinigt und mindestens einen der folgenden Syntheseschritte durchführt:
- Dehydrierung der C1-C2 Bindung der Formel III,
- Hydrierung der exocyclischen Doppelbindung,
- Oxidation der -CH₂OH-Gruppe zum Aldehyd oder zur Carbonsäure, bzw. zum Carbonsäureester,
- Glykosidierung der Hydroxylgruppe am C3-Atom,
oder
(c) die Verbindung der Formel IV 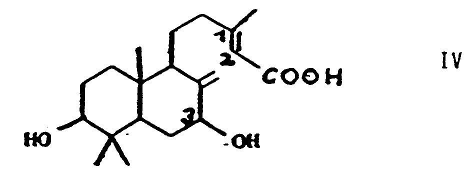 aus Pflanzen der Gattung Ayapana isoliert und aufreinigt und mindestens einen der folgenden Syntheseschritte durchführt:
- Hydrierung der trisubstituierten Doppelbindung,
- Hydrierung der exocyclischen Doppelbindung,
- Veresterung der Carbonsäure,
- Reduktion der Carbonsäure oder des Carbonsäureesters zum Aldehyd oder Alkohol,
- Glykosidierung der Hydroxylgruppe am C3-Atom.

8. Verwendung der Verbindungen der Formel I gemäß Anspruch 1 zur Herstellung von Arzneimitteln mit immunmodulatorischer Wirkung.

9. Verwendung der Verbindungen der Formel I nach Anspruch 8 zur Herstellung von Arzneimitteln zur Stimulation von T-Zellen.

## Patentansprüche (Patentansprüche für folgende(n) Vertragsstaat(en): ES)

1. Verfahren zur Herstellung pharmazeutischer Zubereitungen, dadurch gekennzeichnet, daß man eine Verbindung der Formel I 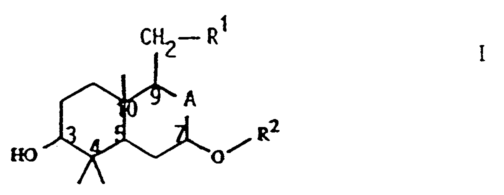 worin
R¹ -CH₂-C(CH₃)=CH-R³ oder -CH₂-CH(CH₃)-CH₂-R³,
R³ -CH₂OH, -CHO, -COOH oder -COOR⁴,
R⁴ Alkyl mit 1 bis 5 C-Atomen,
A
〉CH-CH₃
oder
〉C=CH₂
und
R Pentosen, Hexosen, Disaccharide, Glucosamin oder Galactosamin
bedeuten, und/oder eines ihrer physiologisch unbedenklichen Säureadditionsalze zusammen mit mindestens einem festen, flüssigen oder halbflüssigen Träger- oder Hilfsstoff in eine geeignete Dosierungsform bringt.

2. Verfahren nach Anspruch 1, dadurch gekennzeichnet, daß man eine Verbindung der Formel I einsetzt, worin R Galactose ist.

3. Verfahren nach Anspruch 2, dadurch gekennzeichnet, daß man die Verbindung der Formel Ia einsetzt.

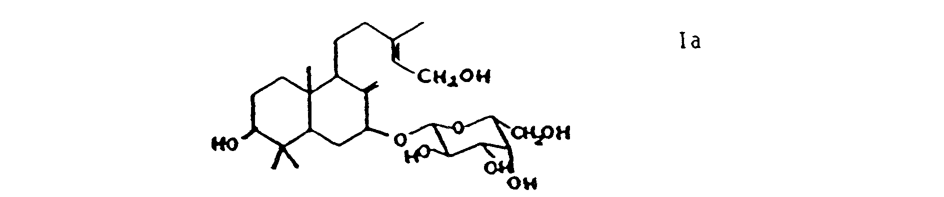

4. Verfahren zur Herstellung der Verbindungen der Formel II 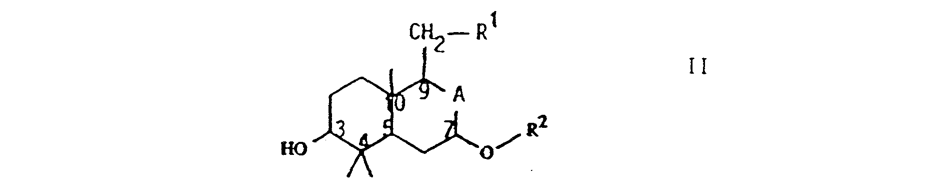 worin
R¹ -CH₂-C(CH₃)=CH-R³ oder -CH₂-CH(CH₃)-CH₂-R³,
R³ -CH₂OH, -CHO, -COOH oder -COOR⁴,
R⁴ Alkyl mit 1 bis 5 C-Atomen,
A
〉CH-CH₃
oder
〉C=CH₂
und
R Pentosen, Hexosen, Disaccharide, Glucosamin oder Galactosamin bedeuten,
mit der Maßgabe, daß nicht gleichzeitig R¹ -CH₂-C(CH₃)=CH-CH₂OH oder -CH₂-C(CH₃)=CH-CHO, A
〉C=CH₂
und R Galactose bedeuten darf,
dadurch gekennzeichnet, daß man wahlweise
(a) die Verbindung der Formel Ia aus Pflanzen der Gattung Acanthospermum isoliert und aufreinigt und mindestens einen der folgenden Syntheseschritte durchführt:
- Hydrierung der trisubstituierten Doppelbindung,
- Hydrierung der exocyclischen Doppelbindung,
- Oxidation der -CH₂OH-Gruppe des Diterpenanteils zum Aldehyd oder zur Carbonsäure, bzw. zum Carbonsäureester,
- Substitution des Galactoserestes durch einen anderen Zucker,
(b) die Verbindung der Formel III 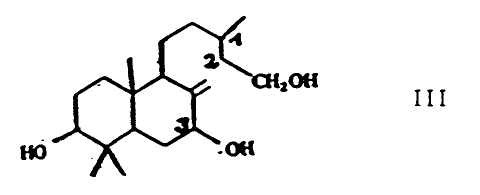 aus Pflanzen der Gattungen Brickellia oder Halimium isoliert und aufreinigt und mindestens einen der folgenden Syntheseschritte durchführt:
- Dehydrierung der C1-C2 Bindung der Formel III,
- Hydrierung der exocyclischen Doppelbindung,
- Oxidation der -CH₂OH-Gruppe zum Aldehyd oder zur Carbonsäure, bzw. zum Carbonsäureester,
- Glykosidierung der Hydroxylgruppe am C3-Atom,
oder
(c) die Verbindung der Formel IV 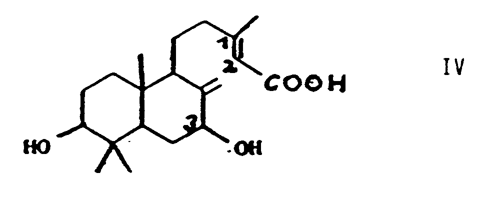 aus Pflanzen der Gattung Ayapana isoliert und aufreinigt und mindestens einen der folgenden Syntheseschritte durchführt:
- Hydrierung der trisubstituierten Doppelbindung,
- Hydrierung der exocyclischen Doppelbindung,
- Veresterung der Carbonsäure,
- Reduktion der Carbonsäure oder des Carbonsäureesters zum Aldehyd oder Alkohol,
- Glykosidierung der Hydroxylgruppe am C3-Atom.

5. Verwendung der Verbindungen der Formel I gemäß anspruch 1 zur Herstellung von Arzneimitteln mit immunmodulatorischer Wirkung.

6. Verwendung der Verbindungen der Formel I nach Anspruch 5 zur Herstellung von Arzneimitteln zur Stimulation von T-Zellen.

## Patentansprüche (Patentansprüche für folgende(n) Vertragsstaat(en): GR)

1. Verfahren zur Herstellung pharmazeutischer Zubereitungen, dadurch gekennzeichnet, daß man eine Verbindung der Formel I 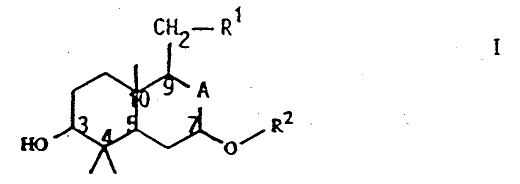 worin
R¹ -CH₂-C(CH₃)=CH-R³ oder -CH₂-CH(CH₃)-CH₂-R³,
R³ -CH₂OH, -CHO, -COOH oder -COOR⁴,
R⁴ Alkyl mit 1 bis 5 C-Atomen,
A
〉CH-CH₃
oder
〉C=CH₂
und
R Pentosen, Hexosen, Disaccharide, Glucosamin oder Galactosamin
bedeuten, und/oder eines ihrer physiologisch unbedenklichen Säureadditionsalze zusammen mit mindestens einem festen, flüssigen oder halbflüssigen Träger- oder Hilfsstoff in eine geeignete Dosierungsform bringt.

2. Verfahren nach Anspruch 1, dadurch gekennzeichnet, daß man eine Verbindung der Formel I einsetzt, worin R Galactose ist.

3. Verfahren nach Anspruch 2, dadurch gekennzeichnet, daß man die Verbindung der Formel Ia einsetzt.

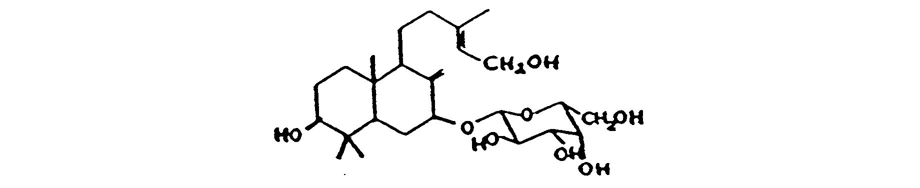

4. Verbindungen der Formel II 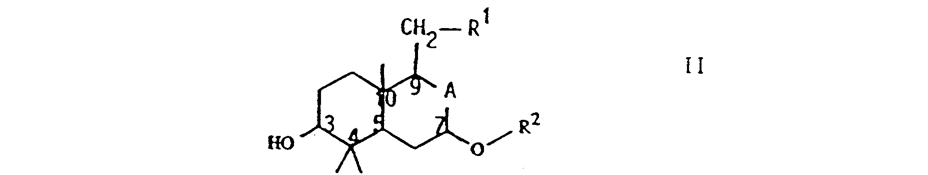 worin
R¹ -CH₂-C(CH₃)=CH-R³ oder -CH₂-CH(CH₃)-CH₂-R³,
R³ -CH₂OH, -CHO, -COOH oder -COOR⁴,
R⁴ Alkyl mit 1 bis 5 C-Atomen,
A
〉CH-CH₃
oder
〉C=CH₂
und
R Pentosen, Hexosen, Disaccharide, Glucosamin oder Galactosamin bedeuten,
mit der Maßgabe, daß nicht gleichzeitig R¹ -CH₂-C(CH₃)=CH-CH₂OH oder -CH₂-C(CH₃)=CH-CHO, A
〉C=CH₂
und R Galactose bedeuten darf.

5. Verfahren zur Herstellung der Verbindungen der Formel II gemäß Anspruch 4, dadurch gekennzeichnet, daß man wahlweise
(a) die Verbindung der Formel Ia aus Pflanzen der Gattung Acanthospermum isoliert und aufreinigt und mindestens einen der folgenden Syntheseschritte durchführt:
- Hydrierung der trisubstituierten Doppelbindung,
- Hydrierung der exocyclischen Doppelbindung,
- Oxidation der -CH₂OH-Gruppe des Diterpenanteils zum Aldehyd oder zur Carbonsäure, bzw. zum Carbonsäureester,
- Substitution des Galactoserestes durch einen anderen Zucker,
(b) die Verbindung der Formel III 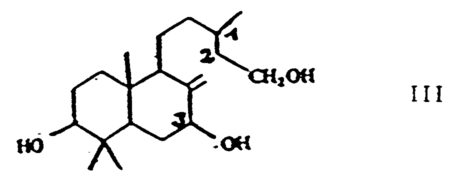 aus Pflanzen der Gattungen Brickellia oder Halimium isoliert und aufreinigt und mindestens einen der folgenden Syntheseschritte durchführt:
- Dehydrierung der C1-C2 Bindung der Formel III,
- Hydrierung der exocyclischen Doppelbindung,
- Oxidation der -CH₂OH-Gruppe zum Aldehyd oder zur Carbonsäure, bzw. zum Carbonsäureester,
- Glykosidierung der Hydroxylgruppe am C3-Atom,
oder
(c) die Verbindung der Formel IV 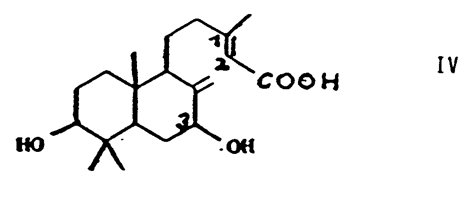 aus Pflanzen der Gattung Ayapana isoliert und aufreinigt und mindestens einen der folgenden Syntheseschritte durchführt:
- Hydrierung der trisubstituierten Doppelbindung,
- Hydrierung der exocyclischen Doppelbindung,
- Veresterung der Carbonsäure,
- Reduktion der Carbonsäure oder des Carbonsäureesters zum Aldehyd oder Alkohol,
- Glykosidierung der Hydroxylgruppe am C3-Atom.

6. Verwendung der Verbindungen der Formel I gemäß Anspruch 1 zur Herstellung von Arzneimitteln mit immunmodulatorischer Wirkung.

7. Verwendung der Verbindungen der Formel I nach Anspruch 6 zur Herstellung von Arzneimitteln zur Stimulation von T-Zellen.

## Claims (Claims for the following Contracting State(s): DE, GB, FR, IT, NL, SE, CH, LU, BE, AT, DK)

1. Medicaments of the formula I 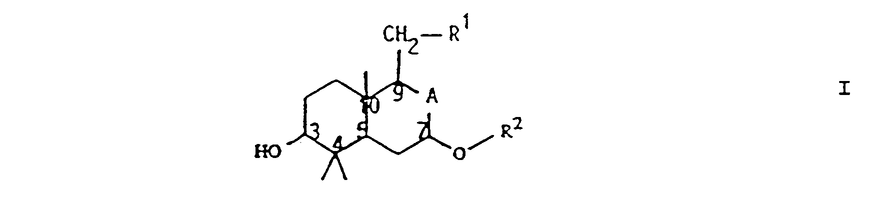 in which
R¹ is - CH₂C(CH₃)=CH-R³ or - CH₂-CH(CH₃)-CH₂-R³,
R³ is -CH₂OH, -CHO, -COOH or -COOR⁴,
R⁴ is alkyl having 1 to 5 C atoms,
A is
〉CH-CH₃
or
〉C=CH₂
and
R is pentoses, hexoses, disaccharides, glucosamine or galactosamine.

2. Medicaments according to Claim 1, in which R is galactose.

3. Medicaments of the formula Ia

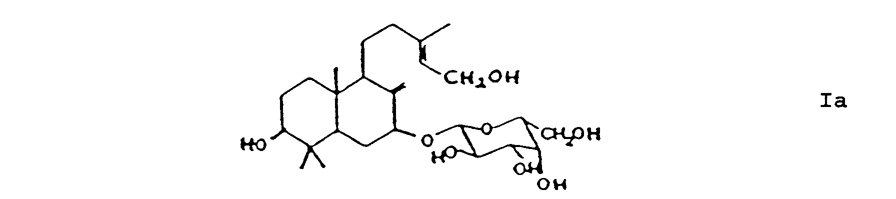

4. Pharmaceutical preparation, characterised in that it contains at least one compound of the formula I according to Claim 1, and/or one of its physiologically acceptable acid addition salts.

5. Process for the production of pharmaceutical preparations, characterised in that a compound of the formula I according to Claim 1, and/or one of its physiologically acceptable acid addition salts is brought into a suitable dose form together with at least one solid, liquid or semi-solid excipient or auxiliary.

6. Compounds of the formula II 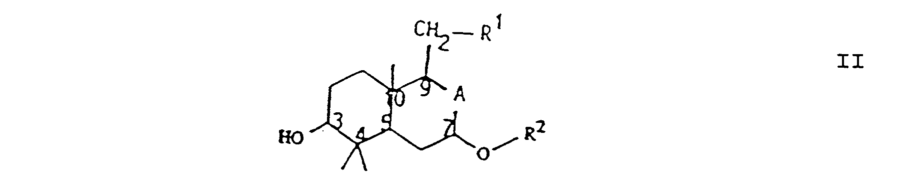 in which
R¹ is -CH₂C(CH₃)=CH-R³ or -CH₂-CH(CH₃)-CH₂-R³,
R³ is -CH₂OH, -CHO, -COOH or -COOR⁴,
R⁴ is alkyl having 1 to 5 C atoms,
A is
〉CH-CH₃
or
〉C=CH₂
and
R is pentoses, hexoses, disaccharides, glucosamine or galactosamine,
with the proviso that R¹ must not be -CHG₂-C(CH₃)=CH-CH₂OH or -CH₂-C(CHG₃)=CH-CHO, A must not be
〉C=CH₂
and R must not be galactose at the same time.

7. Process for the preparation of the compounds of the formula II according to Claim 6, characterised in that, alternatively,
(a) the compound of the formula Ia is isolated from plants of the genus Acanthospermum and purified and at least one of the following synthesis steps is carried out:
- hydrogenation of the trisubstituted double bond,
- hydrogenation of the exocyclic double bond,
- oxidation of the -CH₂OH group of the diterpene component to the aldehyde or to the carboxylic acid, or to the carboxylic acid ester,
- substitution of the galactose residue by another sugar,
(b) the compound of the formula III 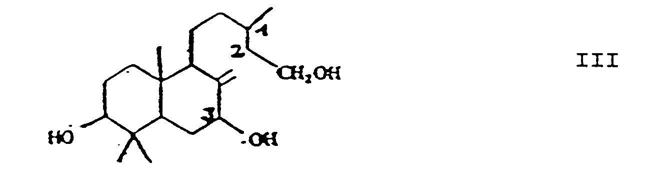 is isolated from plants of the genera Brickellia or Halimium and purified and at least one of the following synthesis steps is carried out:
- dehydration of the C1-C2 bond in the formula III,
- hydrogenation of the exocyclic double bond,
- oxidation of the -CH₂OH group to the aldehyde or to the carboxylic acid, or to the carboxylic acid ester,
- glycosidation of the hydroxyl group on the C3 atom,
or
(c) the compound of the formula IV 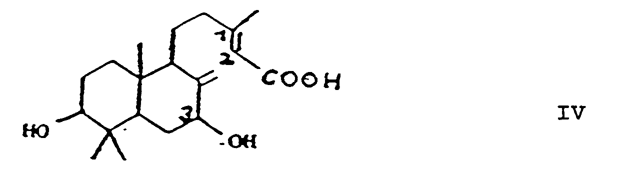 is isolated from plants of the genus Ayapana and purified and at least one of the following synthesis steps is carried out:
- hydrogenation of the trisubstituted double bond,
- hydrogenation of the exocyclic double bond,
- esterification of the carboxylic acid,
- reduction of the carboxylic acid or the carboxylic acid ester to the aldehyde or alcohol
- glycosidation of the hydroxyl group on the C3 atom.

8. Use of the compounds of the formula I according to Claim 1 for the production of medicaments having immunomodulatory action.

9. Use of the compounds of the formula I according to Claim 8 for the production of medicaments for the stimulation of T cells.

## Claims (Claims for the following Contracting State(s): GR)

1. Process for the production of pharmaceutical preparations, characterised in that a compound of the formula I 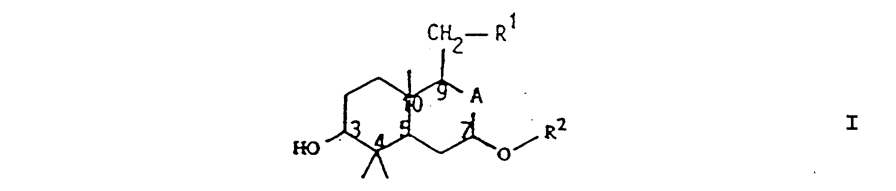 in which
R¹ is -CH₂C(CH₃)=CH-R³ or -CH₂-CH(CH₃)-CH₂-R³,
R³ is -CH₂OH, -CHO, -COOH or -COOR⁴,
R⁴ is alkyl having 1 to 5 C atoms,
A is
〉CH-CH₃
or
〉C=CH₂
and
R is pentoses, hexoses, disaccharides, glucosamine or galactosamine, and/or one of its physiologically acceptable acid addition salts is brought into a suitable dose form together with at least one solid, liquid or semi-solid excipient or auxiliary.

2. Process according to Claim 1, characterised in that a compound of the formula I is employed in which R is galactose.

3. Process according to Claim 2, characterised in that the compound of the formula Ia is used.

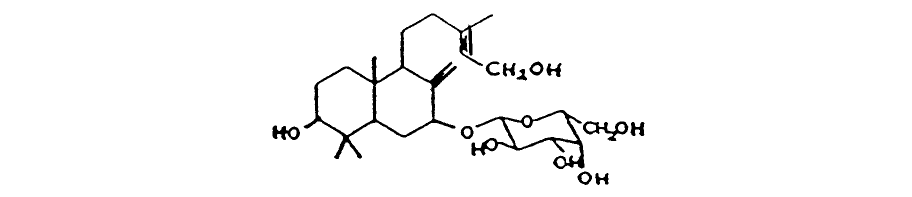

4. Compounds of the formula II 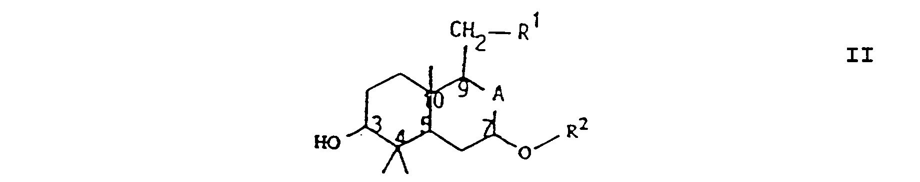 in which
R¹ is -CH₂C(CH₃)=CH-R³ or -CH₂-CH(CH₃)-CH₂-R³,
R³ is -CH₂OH, -CHO, -COOH or -COOR⁴,
R⁴ is alkyl having 1 to 5 C atoms,
A is
〉CH-CH₃
or
〉C=CH₂
and
R is pentoses, hexoses, disaccharides, glucosamine or galactosamine,
with the proviso that R¹ must not be -CHG₂-C(CH₃)=CH-CH₂OH or -CH₂-C(CHG₃)=CH-CHO, A must not be
〉C=CH₂
and R must not be galactose at the same time.

5. Process for the preparation of the compounds of the formula II according to Claim 4, characterised in that, alternatively,
(a) the compound of the formula Ia is isolated from plants of the genus Acanthospermum and purified and at least one of the following synthesis steps is carried out:
- hydrogenation of the trisubstituted double bond,
- hydrogenation of the exocyclic double bond,
- oxidation of the -CH₂OH group of the diterpene component to the aldehyde or to the carboxylic acid, or to the carboxylic acid ester,
- substitution of the galactose residue by another sugar,
(b) the compound of the formula III 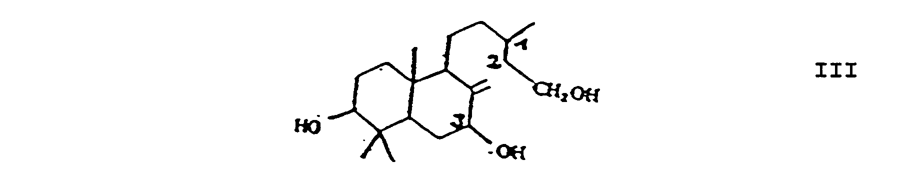 is isolated from plants of the genera Brickellia or Halimium and purified and at least one of the following synthesis steps is carried out:
- dehydration of the C1-C2 bond in the formula III,
- hydrogenation of the exocyclic double bond,
- oxidation of the -CH₂OH group to the aldehyde or to the carboxylic acid, or to the carboxylic acid ester,
- glycosidation of the hydroxyl group on the C3 atom,
or
(c) the compound of the formula IV 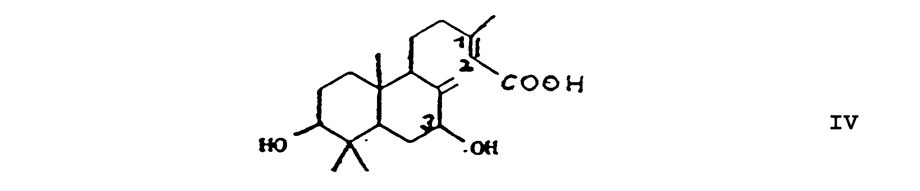 is isolated from plants of the genus Ayapana and purified and at least one of the following synthesis steps is carried out:
- hydrogenation of the trisubstituted double bond,
- hydrogenation of the exocyclic double bond,
- esterification of the carboxylic acid,
- reduction of the carboxylic acid or the carboxylic acid ester to the aldehyde or alcohol,
- glycosidation of the hydroxyl group on the C3 atom.

6. Use of the compounds of the formula I according to Claim 1 for the production of medicaments having immunomodulatory action.

7. Use of the compounds of the formula I according to Claim 6 for the production of medicaments for the stimulation of T cells.

## Claims (Claims for the following Contracting State(s): ES)

1. Process for the production of pharmaceutical preparations, characterized in that a compound of the formula I 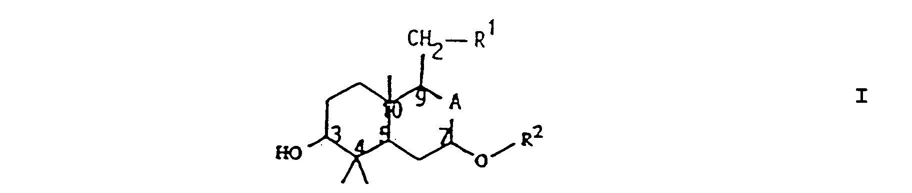 in which
R¹ is -CH₂C(CH₃)=CH-R³ or -CH₂-CH(CH₃)-CH₂-R³,
R³ is -CH₂OH, -CHO, -COOH or -COOR⁴,
R⁴ is alkyl having 1 to 5 C atoms,
A is
〉CH-CH₃
or
〉C=CH₂
and
R is pentoses, hexoses, disaccharides, glucosamine or galactosamine, and/or one of its physiologically acceptable acid addition salts is brought into a suitable dose form together with at least one solid, liquid or semi-solid excipient or auxiliary.

2. Process according to Claim 1, characterized in that a compound of the formula I is used in which R is galactose.

3. Process according to Claim 2, characterized in that the compound of the formula Ia is employed

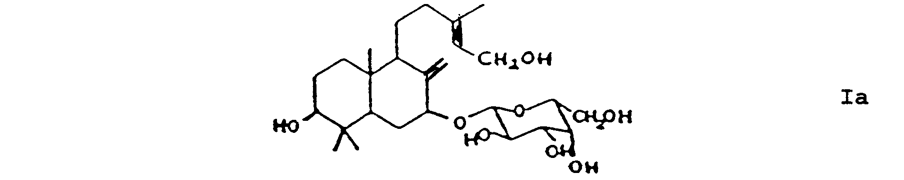

4. Process for the preparation of the compounds of the formula II 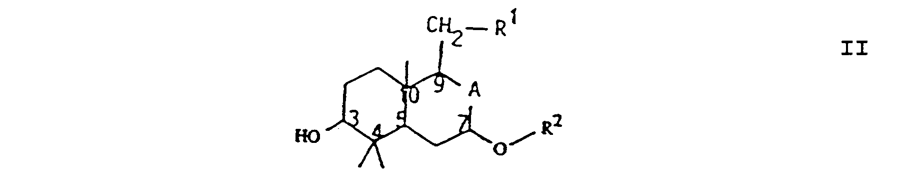 in which
R¹ is - CH₂C(CH₃)=CH-R³ or -CH₂-CH(CH₃)-CH₂-R³,
R³ is -CH₂OH, -CHO, -COOH or -COOR⁴,
R⁴ is alkyl having 1 to 5 C atoms,
A is
〉CH-CH₃
or
〉C=CH₂
and
R is pentoses, hexoses, disaccharides, glucosamine or galactosamine,
with the proviso that R¹ must not be -CHG₂-C(CH₃)=CH-CH₂OH or -CH₂-C(CHG₃)=CH-CHO, A must not be
〉C=CH₂
and R must not be galactose at the same time, characterised in that, alternatively,
(a) the compound of the formula Ia is isolated from plants of the genus Acanthospermum and purified and at least one of the following synthesis steps is carried out:
- hydrogenation of the trisubstituted double bond,
- hydrogenation of the exocyclic double bond,
- oxidation of the -CH₂OH group of the diterpene component to the aldehyde or to the carboxylic acid, or to the carboxylic acid ester,
- substitution of the galactose residue by another sugar,
(b) the compound of the formula III 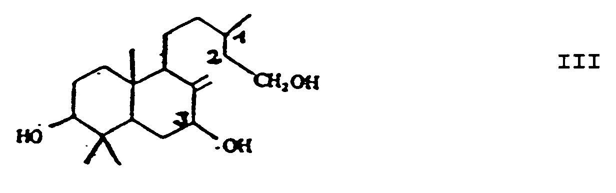 is isolated from plants of the genera Brickellia or Halimium and purified and at least one of the following synthesis steps is carried out:
- dehydration of the C1-C2 bond in the formula III,
- hydrogenation of the exocyclic double bond,
- oxidation of the -CH2OH group to the aldehyde or to the carboxylic acid, or to the carboxylic acid ester,
- glycosidation of the hydroxyl group on the C3 atom,
or
(c) the compound of the formula IV 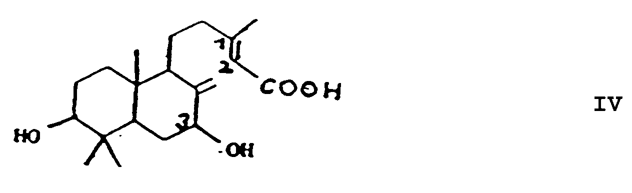 is isolated from plants of the genus Ayapana and purified and at least one of the following synthesis steps is carried out:
- hydrogenation of the trisubstituted double bond,
- hydrogenation of the exocyclic double bond,
- esterification of the carboxylic acid,
- reduction of the carboxylic acid or the carboxylic acid ester to the aldehyde or alcohol
- glycosidation of the hydroxyl group on the C3 atom.

5. Use of the compounds of the formula I according to Claim 1 for the production of medicaments having immunomodulatory action.

6. Use of the compounds of the formula I according to Claim 5 for the production of medicaments for the stimulation of T cells.

## Revendications (Revendications pour l'(les) Etat(s) contractant(s) suivant(s): DE, GB, FR, IT, NL, SE, CH, LU, BE, AT, DK)

1. Médicament de formule I 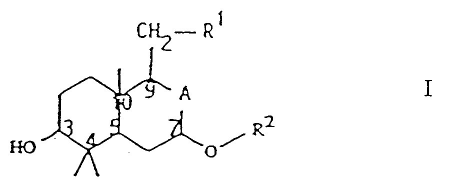 dans laquelle
R¹ représente -CH₂-C(CH₃)=CH-R³ ou -CH₂-CH(CH₃)-CH₂-R³,
R³ représente -CH₂OH, -CHO, -COOH ou -COOR⁴,
R⁴ représente un groupe alkyle en C1-C5,
A représente
〉CH-CH₃
ou
〉C=CH₂
et
R représente des pentoses, des hexoses, des disaccharides, la glucosamine ou la galactosamine.

2. Médicament selon revendication 1, pour lequel R représente le galactose.

3. Médicament de formule Ia

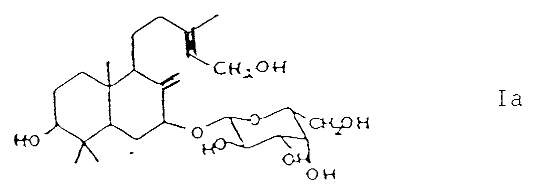

4. Composition pharmaceutique caractérisée en ce qu'elle contient au moins un composé de formule I selon revendication 1 et/ou l'un de ses sels formé par addition avec un acide acceptable pour l'usage pharmaceutique.

5. Procédé de préparation de compositions pharmaceutiques, caractérisé en ce que l'on met un composé de formule I selon revendication 1 et/ou l'un de ses sels formé par addition avec un acide acceptable pour l'usage pharmaceutique sous une forme de dosage appropriée avec au moins un véhicule ou produit auxiliaire solide, liquide ou semi-liquide.

6. Composés de formule II 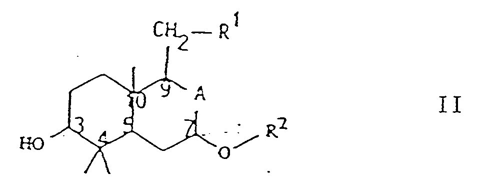 dans laquelle
R¹ représente -CH₂-C(CH₃)=CH-R³ ou -CH₂-CH(CH₃)-CH₂-R³,
R³ représente -CH₂OH, -CHO, -COOH ou -COOR⁴,
R⁴ représente un groupe alkyle en C1-C5,
A représente
〉CH-CH₃
ou
〉C=CH₂
et
R représente des pentoses, des hexoses, des disaccharides, la glucosamine ou la galactosamine,
àl'exclusion des composés pour lesquels, simultanément, R¹ représente -CH₂-C(CH₃)=CH-CH₂OH ou -CH₂-C(CH₃)=CH-CHO, A représente
〉C=CH₂
et R le galactose.

7. Procédé de préparation des composés de formule II de la revendication 6, caractérisé en ce que, à volonté,
(a) on isole le composé de formule la de végétaux du genre Acanthospermum et on la purifie puis on procède à au moins un des stades de synthèse suivants :
- hydrogénation de la double liaison trisubstituée,
- hydrogénation de la double liaison exocyclique,
- oxydation du groupe -CH₂OH de la partie diterpène en aldéhyde ou en acide carboxylique ou en ester d'acide carboxylique,
- remplacement du radical de galactose par un autre sucre,
(b) on isole le composé de formule III 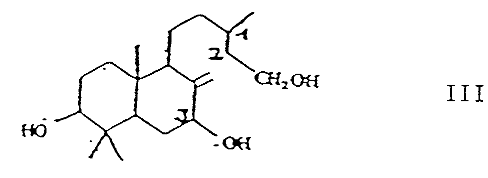 de végétaux des genres Brickellia ou Halimium et on le purifie et on procède à au moins un des stades de synthèse suivants :
- déshydrogénation de la liaison C1-C2 de la formule III,
- hydrogénation de la double liaison exocyclique,
- oxydation du groupe -CH₂OH en aldéhyde ou en acide carboxylique ou en ester d'acide carboxylique,
- glycosidation du groupe hydroxy sur l'atome C3,
(c) on isole le composé de formule IV 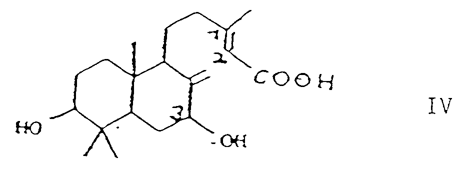 de végétaux du genre Ayapana et on le purifie puis on procède à au moins un des stades de synthèse suivants :
- hydrogénation de la double liaison trisubstituée,
- hydrogénation de la double liaison exocyclique,
- estérification de l'acide carboxylique,
- réduction de l'acide carboxylique ou de l'ester d'acide carboxylique en aldéhyde ou alcool,
- glycosidation du groupe hydroxy sur l'atome C3.

8. Utilisation des composés de formule I de la revendication 1 pour la préparation de médicaments à activité immunomodulatrice.

9. Utilisation des composés de formule I de la revendication 8 pour la préparation de médicaments destinés à la stimulation des cellules T.

## Revendications (Revendications pour l'(les) Etat(s) contractant(s) suivant(s): GR)

1. Procédé de préparation de compositions pharmaceutiques, caractérisé en ce que l'on met un composé de formule I 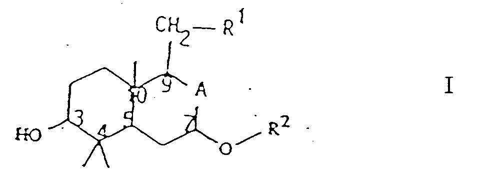 dans laquelle
R¹ représente -CH₂-C(CH₃)=CH-R³ ou -CH₂-CH(CH₃)-CH₂-R³,
R³ représente -CH₂OH, -CHO, -COOH ou -COOR₄,
R⁴ représente un groupe alkyle en C1-C5,
A représente
〉CH-CH₃
ou
〉C=CH₂
et
R représente des pentoses, des hexoses, des disaccharides, la glucosamine ou la galactosamine,
et/ou l'un de ses sels formé par addition avec un acide acceptable pour l'usage pharmaceutique,
sous une forme de dosage appropriée avec au moins un véhicule ou produit auxiliaire solide, liquide ou semi-liquide.

2. Procédé selon revendication 1, caractérisé en ce que l'on utilise un composé de formule I dans laquelle R représente le galactose.

3. Procédé selon revendication 2, caractérisé en ce que l'on utilise le composé de formule la

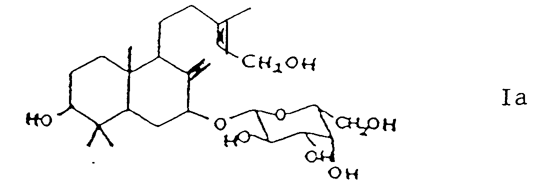

4. Composés de formule II 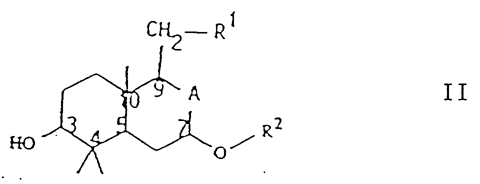 dans laquelle
R¹ représente -CH₂-C(CH₃)=CH-R³ ou -CH₂-CH(CH₃)-CH₂-R³,
R³ représente -CH₂OH, -CHO, -COOH ou -COOR⁴,
R⁴ représente un groupe alkyle en C1-C3,
A représente
〉CH-CH₃
ou
〉C=CH₂
et
R représente des pentoses, des hexoses, des disaccharides, la glucosamine ou la galactosamine,
àl'exclusion des composés pour lesquels, simultanément, R¹ représente -CH₂-C(CH₃)=CH-CH₂OH ou -CH₂-C(CH₃)=CH-CHO, A représente
〉C=CH₂
et R le galactose.

5. Procédé de préparation des composés de formule II de la revendication 4, caractérisé en ce que, au choix
(a) on isole le composé de formule la de végétaux du genre Acanthospermum et on le purifie puis on procède à au moins un des stades de synthèse suivants :
- hydrogénation de la double liaison trisubstituée,
- hydrogénation de la double liaison exocyclique,
- oxydation du groupe -CH₂OH de la partie diterpène en aldéhyde ou en acide carboxylique ou en ester d'acide carboxylique,
- remplacement du radical de galactose par un autre sucre,
(b) on isole le composé de formule III 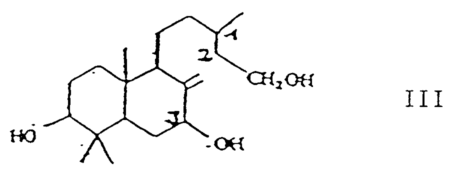 de végétaux des genres Brickellia ou Halimium et on le purifie puis on procède à au moins un des stades de synthèse suivants :
- déshydrogénation de la liaison C1-C2 de la formule III,
- hydrogénation de la double liaison exocyclique,
- oxydation du groupe -CH₂OH en aldéhyde ou en acide carboxylique ou en ester d'acide carboxylique,
- glycosidation du groupe hydroxy sur l'atome C3, ou bien
(c) on isole le composé de formule IV 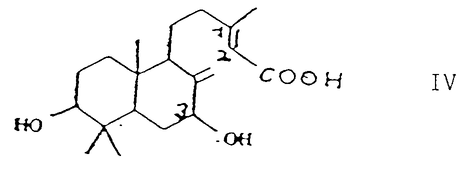 de végétaux du genre Ayapana et on le purifie puis on procède à au moins un des stades de synthèse suivants :
- hydrogénation de la double liaison trisubstituée,
- hydrogénation de la double liaison exocyclique,
- estérification de l'acide carboxylique,
- réduction de l'acide carboxylique ou de l'ester d'acide carboxylique en aldéhyde ou alcool,
- glycosidation du groupe hydroxy sur l'atome C3.

6. Utilisation des composés de formule I de la revendication 1, pour la préparation de médicaments à activité immunomodulatrice.

7. Utilisation des composés de formule I de la revendication 6, pour la préparation de médicaments servant à stimuler les cellules T.

## Revendications (Revendications pour l'(les) Etat(s) contractant(s) suivant(s): ES)

1. Procédé de préparation de compositions pharmaceutiques, caractérisé en ce que l'on met un composé de formule I 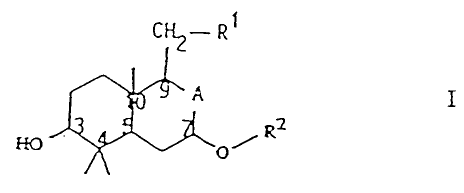 dans laquelle
R¹ représente -CH₂-C(CH₃)=CH-R³ ou -CH₂-CH(CH₃)-CH₂-R³,
R³ représente -CH₂OH, -CHO, -COOH ou -COOR⁴,
R⁴ représente un groupe alkyle en C1-C5,
A représente
〉CH-CH₃
ou
〉C=CH₂
et
R représente des pentoses, des hexoses, des disaccharides, la glucosamine ou la galactosamine,
et/ou l'un de ses sels formé par addition avec un acide acceptable pour l'usage pharmaceutique,
sous une forme de dosage appropriée avec au moins un véhicule ou produit auxiliaire solide, liquide ou semi-liquide.

2. Procédé selon revendication 1, caractérisé en ce que l'on utilise un composé de formule I dans laquelle R représente le galactose.

3. Procédé selon revendication 2, caractérisé en ce que l'on utilise le composé de formule la

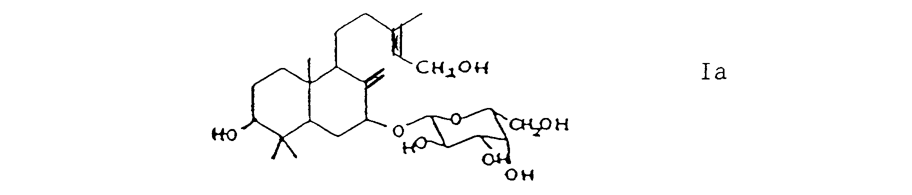

4. Procédé de préparation des composés de formule II 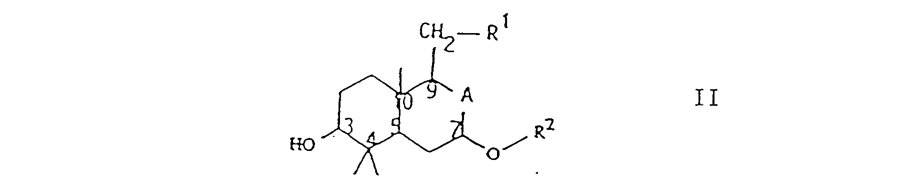 dans laquelle
R¹ représente -CH₂-C(CH₃)=CH-R³ ou -CH₂-CH(CH₃)-CH₂-R³,
R³ représente -CH₂OH, -CHO, -COOH ou -COOR⁴,
R⁴ représente un groupe alkyle en C1-C5,
A représente
〉CH-CH₃
ou
〉C=CH₂
et
R représente des pentoses, des hexoses, des disaccharides, la glucosamine ou la galactosamine,
à l'exclusion des composés pour lesquels, simultanément, R¹ représente -CH₂-C(CH₃)-CH-CH₂OH ou -CH₂-C(CH₃)=CH-CHO, A représente
〉C=CH₂
représente le galactose, caractérisé en ce que, à volonté,
(a) on isole le composé de formule la de végétaux du genre Acanthospermum et on le purifie puis on procède à au moins un des stades de synthèse suivants :
- hydrogénation de la double liaison trisubstituée,
- hydrogénation de la double liaison exocyclique,
- oxydation du groupe -CH₂OH de la partie diterpène en aldéhyde ou en acide carboxylique ou en ester d'acide carboxylique,
- remplacement du radical de galactose par un autre sucre,
(b) on isole le composé de formule III 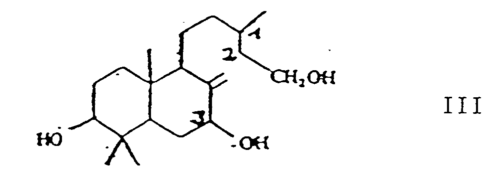 de végétaux des genres Brickellia ou Halimium et on le purifie puis on procède à au moins un des stades de synthèse suivants :
- déshydrogénation de la liaison C1-C2 de la formule III,
- hydrogénation de la double liaison exocyclique,
- oxydation du groupe -CH₂OH en aldéhyde ou en acide carboxylique ou en ester d'acide carboxylique,
- glycosidation du groupe hydroxy sur l'atome C3,
ou bien
(c) on isole le composé de formule IV 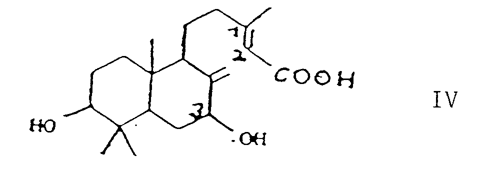 de végétaux du genre Ayapana et on le purifie puis on procède à au moins un des stades de synthèse suivants :
- hydrogénation de la double liaison trisubstituée,
- hydrogénation de la double liaison exocyclique,
- estérification de l'acide carboxylique,
- réduction de l'acide carboxylique ou de l'ester d'acide carboxylique en aldéhyde ou alcool,
- glycosidation du groupe hydroxy sur l'atome C3.

5. Utilisation des composés de formule I de la revendication 1 pour la préparation de médicaments à activité immunomodulatrice.

6. Utilisation des composés de formule I de la revendication 5 pour la préparation de médicaments servant à stimuler les cellules T.
